# EUROPÄISCHE PATENTSCHRIFT

(11) **EP 1 147 228 B1**
(45) Veröffentlichungstag und Bekanntmachung des Hinweises auf die Patenterteilung: **15.10.2003**
(21) Anmeldenummer: 00908971.5
(22) Anmeldetag: 27.01.2000
(51) Int. Cl.: C12Q 1/68

(54) **VERFAHREN ZUR IDENTIFIKATION VON CYTOSIN-METHYLIERUNGSMUSTERN IN GENOMISCHEN DNA-PROBEN**
METHOD OF IDENTIFYING CYTOSINE METHYLATION PATTERNS IN GENOMIC DNA SAMPLES
PROCEDE D'IDENTIFICATION DE MODELES DE METHYLATION DE CYTOSINE DANS DES ECHANTILLONS D'ADN GENOMIQUES

(30) Priorität: 29.01.1999 DE 19905082
(43) Veröffentlichungstag der Anmeldung: 24.10.2001
(73) Patentinhaber: Epigenomics AG, 10435 Berlin (DE)
(72) Erfinder: OLEK, Alexander, D-10781 Berlin (DE)
(74) Vertreter: Schubert, Klemens, Dr.
(86) Internationale Anmeldenummer: DE0000288
(87) Internationale Veröffentlichungsnummer: WO00044934

(56) Entgegenhaltungen:
- WO-A-97/37041
- DE-A- 19 543 065
- US-A- 5 605 798
- REIN ET AL: "Identifying 5-methylcytosine and related modifications in DNA genomes" NUCLEIC ACIDS RESEARCH, Bd. 26, Nr. 10, 1998, XP002143106 in der Anmeldung erwähnt
- PAUL C L ET AL: "Cytosine methylation: quantitation by automated genomic sequencing and GENESCAN analysis." BIOTECHNIQUES, (1996 JUL) 21 (1) 126-33. , XP002143107

## Beschreibung

Die Erfindung betrifft ein Verfahren zur Identifikation von Cytosin-Methylierungsmustern in genomischen DNA-Proben.

Die genetische Information, die durch vollständige Sequenzierung genomischer DNA als Basenabfolge erhalten wird, beschreibt das Genom einer Zelle nur unvollständig. 5-Methylcytosin-Nukleobasen, die durch reversible Methylierung von DNA in der Zelle entstehen, sind ein epigenetischer Informationsträger und dienen beispielsweise zur Regulation von Promotoren. Der Methylierungszustand eines Genoms repräsentiert den gegenwärtigen Status der Genexpression, ähnlich wie ein mRNA Expressionsmuster.

5-Methylcytosin ist die häufigste kovalent modifizierte Base in der DNA eukaryotischer Zellen. Sie spielt beispielsweise eine Rolle in der Regulation der Transkription, genomischem Imprinting und in der Tumorgenese. Die Identifizierung von 5-Methylcytosin als Bestandteil genetischer Information ist daher von erheblichem Interesse. 5-Methylcytosin-Positionen können jedoch nicht durch Sequenzierung identifiziert werden, da 5-Methylcytosin das gleiche Basenpaarungsverhalten aufweist wie Cytosin. Darüberhinaus geht bei einer PCR-Amplifikation die epigenetische Information, die die 5-Methylcytosine tragen, vollständig verloren.

Es sind mehrere Verfahren bekannt, die diese Probleme zu lösen versuchen. Meist wird eine chemische Reaktion oder enzymatische Behandlung der genomischen DNA durchgeführt, in deren Folge sich die Cytosin- von den Methylcytosin-Basen unterscheiden lassen. Eine gängige Methode ist die Umsetzung von genomischer DNA mit Bisulfit, die nach alkalischer Hydrolyse in zwei Schritten zu einer Umwandlung der Cytosin Basen in Uracil führt (Shapiro, R., Cohen, B., Servis, R. Nature 227, 1047 (1970). 5-Methylcytosin bleibt unter diesen Bedingungen unverändert. Die Umwandlung von C in U führt zu einer Veränderung der Basensequenz, aus der sich durch Sequenzierung nun die ursprünglichen 5-Methylcytosine ermitteln lassen (nur diese liefern noch eine Bande in der C-Spur).

Eine Übersicht über weitere bekannte Möglichkeiten, 5-Methylcytosine nachzuweisen, ist beispielsweise dem folgenden Übersichtsartikel zu entnehmen: Rein, T., DePamphilis, M. L., Zorbas, H., Nucleic Acids Res. 26, 2255 (1998).

Die Bisulphit-Technik wird bisher bis auf wenige Ausnahmen (z.B. Zeschnigk, M. et al., Eur. J. Hum. Gen. 5, 94-98; Kubota T. et al., Nat. Genet. 16, 16-17) nur in der Forschung angewendet. Immer aber werden kurze, spezifische Stücke eines bekannten Gens nach einer Bisulphit-Behandlung amplifiziert und entweder komplett sequenziert (Olek, A. and Walter, J., Nat. Genet. 17, 275-276) oder einzelne Cytosin-Positionen durch eine "Primer-Extension-Reaktion" (Gonzalgo, M. L. and Jones, P.A., Nucl. Acids. Res. 25, 2529-2531) oder Enzymschnitt (Xiong, Z. and Laird, P.W., Nucl. Acids. Res. 25, 2532-2534) nachgewiesen. Alle diese Referenzen stammen aus dem Jahre 1997. Das Konzept, komplexe Methylierungsmuster zur Korrelation mit phänotypischen Daten komplexer genetischer Erkrankungen zu verwenden, ist lediglich in DE-19543065 A1 erwähnt. So wird hierin zum Beispiel die eigentliche Detektion nicht über die Analyse der Hybrisierung von Nukleinsäure-Sonden im Massenspektrometer durchgeführt.

Nicht immer ist es erforderlich, tatsächlich die gesamte Sequenz eines Gens oder Genabschnitts zu ermitteln, wie dies bei einer Sequenzierung das Ziel ist. Dies ist insbesondere dann der Fall, wenn wenige 5-Methylcytosin-Positionen innerhalb einer längeren Basensequenz für eine Vielzahl von unterschiedlichen Proben abzutasten sind. Die Sequenzierung liefert hier in großem Umfang redundante Information und ist zudem sehr teuer. Dies ist auch schon dann der Fall, wenn die Sequenz bereits bekannt ist und ausschließlich Methylierungspositionen dargestellt werden sollen. Auch ist es denkbar, daß in einigen Fällen überhaupt nur die Unterschiede im Methylierungsmuster zwischen verschiedenen genomischen DNA-Proben von Interesse sind und daß auf die Ermittlung einer Vielzahl übereinstimmender methylierter Positionen verzichtet werden kann. Für die hier angeführten Fragestellungen existiert bislang kein Verfahren, das ohne Sequenzierung jeder einzelnen Probe, kostengünstig die gewünschten Ergebnisse liefert.

Sequenzinformation muß auch umso mehr immer weniger neu ermittelt werden, da die Genomprojekte, deren Ziel die vollständige Sequenz verschiedener Organismen ist, zügig voranschreiten. Vom menschlichen Genom sind zwar derzeit erst etwa 5 % fertig sequenziert, jedoch kommen jetzt, weil andere Genomprojekte dem Ende zuneigen und dadurch Sequenzierressourcen frei werden, jedes Jahr weitere 5 % dazu. Mit der Vervollständigung der Sequenzierung des menschlichen Genoms wird bis zum Jahre 2006 gerechnet.

Matrix-assistierte Laser Desorptions/Ionisations Massenspektrometrie (MALDI) ist eine neue, sehr leistungsfähige Entwicklung für die Analyse von Biomolekülen (Karas, M. and Hillenkamp, F. 1988. Laser desorption ionization of proteins with molecular masses exceeding 10.000 daltons. Anal. Chem. 60: 2299-2301). Ein Analytmolekül wird in eine im UV absorbierende Matrix eingebettet. Durch einen kurzen Laserpuls wird die Matrix ins Vakuum verdampft und das Analyt so unfragmentiert in die Gasphase befördert. Eine angelegte Spannung beschleunigt die Ionen in ein feldfreies Flugrohr. Auf Grund ihrer verschiedenen Massen werden Ionen unterschiedlich stark beschleunigt. Kleinere Ionen erreichen den Detektor früher als größere. Die Flugzeit wird in die Masse der Ionen umgerechnet.

Technische Neuerungen der Hardware haben die Methode signifikant verbessert. Erwähnenswert ist hierbei die "delayed extraction"-Methode (DE). Für DE wird die Beschleunigungsspannung mit einer Verzögerung zum Laserpuls eingeschaltet und dadurch eine verbesserte Auflösung der Signale erreicht, weil die Zahl der Stöße verringert wird.

MALDI eignet sich ausgezeichnet zur Analyse von Peptiden und Proteinen. Für Nukleinsäuren ist die Empfindlichkeit etwa 100-mal schlechter als für Peptide und nimmt mit zunehmender Fragmentgröße überproportional ab. Der Grund dafür liegt darin, daß für die Ionisation von Peptiden und Proteinen lediglich ein einziges Proton eingefangen werden muß. Für Nukleinsäuren, die ein vielfach negativ geladenes Rückgrat haben, ist der Ionisationsprozeß durch die Matrix wesentlich ineffizienter. Für MALDI spielt die Wahl der Matrix eine eminent wichtige Rolle. Für die Desorption von Peptiden sind einige sehr leistungsfähige Matrices gefunden worden, die eine sehr feine Kristallisation ergeben. Für DNA sind zwar inzwischen einige geeignete Matrices gefunden worden, jedoch wurde dadurch der Empfindlichkeitsunterschied nicht verringert. Phosphorothioatnukleinsäuren, bei denen die gewöhnlichen Phosphate des Rückgrats durch Thiophosphate substituiert sind, lassen sich durch einfache Alkylierungschemie in eine ladungsneutrale DNA umwandeln.

Die Kopplung eines "charge tags" an diese modifizierte DNA resultiert in der Steigerung der Empfindlichkeit in den gleichen Bereich wie er für Peptide gefunden wird. Durch diese Modifikationen hat sich auch die Möglichkeit eröffnet, ähnliche Matrices, wie sie für die Desorption von Peptiden verwendet werden, zu nutzen. Ein weiterer Vorteil von charge tagging ist die erhöhte Stabilität der Analyse gegen Verunreinigungen, die den Nachweis unmodifizierter Substrate stark erschweren. PNAs und Methylphosphonatoligonukleotide sind mit MALDI untersucht worden und lassen sich so analysieren.

Derzeit ist diese Technologie in der Lage, im Massenbereich von 1.000 bis 4.000 Da, Moleküle mit einer Massendifferenz von 1 Da zu unterscheiden. Durch die natürliche Verteilung von Isotopen sind die meisten Biomoleküle jedoch schon etwa 5 Da breit. Technisch ist diese massenspektrometrische Methode also vorzüglich für die Analyse von Biomolekülen geeignet. Vernünftigerweise müssen zu analysierende Produkte, die unterschieden werden sollen, also mindestens 5 Da auseinander liegen. In diesem Massenbereich könnten also 600 Moleküle unterschieden werden.

Ein Array mit vielen tausend Ziel-DNAs kann auf einer Festphase immobilisiert und anschließend können alle Ziel-DNAs gemeinsam auf das Vorhandensein einer Sequenz mittels einer Sonde (Nukleinsäure mit komplementärer Sequenz) untersucht werden.

Eine Übereinstimmung in der Ziel-DNA mit der Sonde kommt durch eine Hybridisation der zwei Teile miteinander zustande. Sonden können beliebige Nukleinsäuresequenzen von beliebiger Länge sein. Es existieren verschiedene Verfahren für die Auswahl von optimalen Bibliotheken von Sondensequenzen, welche minimal miteinander überlappen. Sondensequenzen können auch gezielt zusammengestellt werden, um bestimmte Ziel-DNA Sequenzen aufzufinden. Oligofingerprinting ist ein Ansatz, bei welchem diese Technologie zum Einsatz kommt. Eine Bibliothek von Ziel-DNAs wird mit kurzen Nukleinsäuresonden abgetastet. Meist sind hier die Sonden nur 8-12 Basen lang. Es wird jeweils eine Sonde auf einmal an eine auf einer Nylonmembran immobilisierte Ziet-DNA-Bibliothek hybridisiert. Die Sonde ist radioaktiv markiert und die Hybridisierung wird anhand der Lokalisierung der Radioaktivität beurteilt. Für die Abtastung eines immobilisierten DNA-Arrays sind auch schon fluoreszent markierte Sonden verwendet worden.

US 5,605,798 beschreibt die Abtastung von über Hybridisierung immobilisierten Ziel-Nukleinsäuren mit Nukleinsäuresonden und Massenspektrometrie. Hierbei wird jedoch weder gezielt eine Identifikation von Methylierungsmustern durchgeführt, noch werden modifizierte Nukleinsäuren (z. B. PNAs, Charge Tags) eingesetzt, noch wird eine Genomamplifikation durchgeführt.

Die US 5,605,798 offenbart also ein Verfahren zur Detektion von bestimmten Nukleinsäuresequenzen in einer Probe mit Hilfe einer Amplifikation und anschließender Massenspektrometrie als Sequenzierungsmethode. Hierbei muss die Sequenz der zu bestimmenden Nukleinsäuren bekannt sein, damit die hybridisierenden Primer hergestellt werden können. In diesem Dokument werden keine CpG-enthaltenden Sonden verwendet. Es gibt auch keinen Hinweis darauf, dass Massenspektrometrie für die Identifikation von Methylierungsmustern geeignet ist.

In Biotechniques, Band 21, Nr. 1, 1996, Seiten 126-133 und in WO-A-9737041 werden klassische Sequenzierungstechniken beschrieben, welche eine Primerverlängerung wie bei der Sanger-Sequenzierung nutzen.

Als Sonden kommen jegliche Moleküle in Frage, die sequenzspezifisch mit einer Ziel-DNA wechselwirken können. Am gängigsten sind Oligodeoxyribonukleotide. Jedoch bietet sich jede Modifikation von Nukleinsäuren an, z.B. Peptide Nucleic Acids (PNA), Phosphorothioatoligonukleotide oder Methylphosphonatoligonukleotide. Die Spezifität einer Sonde ist sehr wesentlich. Phosphorothioatoligonukleotide sind nicht allzugut geeignet, da ihre Struktur durch die Schwefelatome und dadurch die Hybridisierungseigenschaft gestört ist. Ein Grund hierfür könnte sein, daß Phosphorothioatoligonukleotide normalerweise nicht diastereomerenrein synthetisiert werden. Bei Methylphosphonatoligonukleotiden besteht ein ähnliches Pro-blem, jedoch werden diese Oligonukleotide vermehrt diastereomerenrein synthetisiert. Ein wesentlicher Unterschied dieser Modifikation ist das ungeladene Rückgrat, welches zu einer verminderten Abhängigkeit der Hybridisation von Puffersalzen und insgesamt durch die geringere Abstoßung zu höherer Affinität führt. Peptide Nucleic Acids haben ebenfalls ein ungeladenes Rückgrat, welches gleichzeitig chemisch sehr stark von der gängigen Zucker-Phosphat Struktur des Rückgrats in Nukleinsäuren abweicht. Das Rückgrat einer PNA hat eine Amidsequenz anstelle des Zucker-Phosphat Rückgrats gewöhnlicher DNA. PNA hybridisiert sehr gut mit DNA komplementärer Sequenz. Die Schmelztemperatur eines PNA/DNA-Hybrids ist höher als die des entsprechenden DNA/DNA-Hybrids und wiederum ist die Abhängigkeit der Hybridisierung von Puffersalzen relativ gering.

Kombinatorische Synthesen, d.h. die Herstellung von Substanzbibliotheken ausgehend von einem Gemisch von Vorstufen, werden sowohl auf fester als auch in flüssiger Phase durchgeführt. Vor allem die kombinatorische Festphasensynthese hat sich frühzeitig etabliert, da in diesem Fall die Abtrennung von Nebenprodukten besonders einfach ist. Nur die an den Support gebundenen Zielverbindungen werden in einem Waschschritt zurückbehalten und am Ende der Synthese durch das gezielte Spalten eines Linkers isoliert. Diese Technik erlaubt auf einfachem Wege die gleichzeitige Synthese einer Vielzahl verschiedener Verbindungen an einer Festphase und somit den Erhalt von chemisch "reinen" Substanzbibliotheken.

Daher sind die Verbindungsklassen, die auch in nicht kombinatorischen, konventionellen Synthesen auf einer Festphase synthetisiert werden, der kombinatorischen Chemie besonders leicht zugänglich und werden demzufolge auch breit verwendet. Dies trifft vor allem auf Peptid-, Nukleinsäure- und PNA-Bibliotheken zu.

Die Synthese von Peptiden erfolgt durch Binden der ersten N-geschützten Aminosäure (z.B. Boc) an den Support, nachfolgende Entschützung und Reaktion der zweiten Aminosäure mit der freigewordenen NH2-Gruppe der ersten. Nicht reagierte Aminofunktionen werden in einem weiteren "Capping" Schritt einer Weiterreaktion im nächsten Synthesezyklus entzogen. Die Schutzgruppe an der Aminofunktion der zweiten Aminosäure wird entfernt und der nächste Baustein kann gekoppelt werden. Zur Synthese von Peptidbibliotheken wird ein Gemisch von Aminosäuren in einem oder mehreren Schritten verwendet. Die Synthese von PNA und PNA-Bibliotheken erfolgt sinngemäß.

Nukleinsäure-Bibliotheken werden meist durch Festphasensynthese mit Gemischen verschiedener Phosphoramidit-Nukleoside erhalten. Dies kann auf kommerziell erhältlichen DNA-Synthesizern ohne Veränderungen in den Syntheseprotokollen durchgeführt werden.

Verschiedene Arbeiten zur kombinatorischen Synthese von PNA Bibliotheken sind publiziert worden. Diese Arbeiten behandeln den Aufbau von kombinatorischen Sequenzen, d.h. die Synthese von PNAs in denen einzelne, spezifische Basen in der Sequenz durch degenerierte Basen ersetzt werden und dadurch zufällige Sequenzvarianz erreicht wird.

Die Verwendung massenspektrometrischer Methoden für die Analyse kombinatorischer Bibliotheken ist mehrfach beschrieben worden.

Es existieren verschiedene Verfahren um DNA zu immobilisieren. Das bekannteste Verfahren ist die Festbindung einer DNA, welche mit Biotin funktionalisiert ist, an eine Streptavidin-beschichtete Oberfläche. Die Bindungsstärke dieses Systems entspricht einer kovalenten chemischen Bindung ohne eine zu sein. Um eine Ziel-DNA kovalent an eine chemisch vorbereitete Oberfläche binden zu können, bedarf es einer entsprechenden Funktionalität der Ziel-DNA. DNA selbst besitzt keine Funktionalisierung, die geeignet ist. Es gibt verschiedene Varianten in eine Ziel-DNA eine geeignete Funktionalisierung einzuführen: Zwei leicht zu handhabende Funktionalisierungen sind primäre, aliphatische Amine und Thiole. Solche Amine werden quantitativ mit N-Hydroxy-succinimidestern umgesetzt und Thiole reagieren unter geeigneten Bedingungen quantitativ mit Alkyliodiden. Eine Schwierigkeit besteht im Einführen einer solchen Funktionalisierung in eine DNA. Die einfachste Variante ist die Einführung durch einen Primer einer PCR. Gezeigte Varianten benützen 5'-modifizierte Primer (NH2 und SH) und einen bifunktionalen Linker.

Ein wesentlicher Bestandteil der Immobilisierung auf einer Oberfläche ist ihre Beschaffenheit. Bis jetzt beschriebene Systeme sind hauptsächlich aus Silizium oder Metall (magnetic beads). Eine weitere Methode zur Bindung einer Ziel-DNA basiert darauf, eine kurze Erkennungssequenz (z.B. 20 Basen) in der Ziel-DNA zur Hybridisierung an ein oberflächenimmobilisiertes Oligonukleotid zu verwenden.

Es sind auch enzymatische Varianten zur Einführung von chemisch aktivierten Positionen in eine Ziel-DNA beschrieben worden. Hier wird an einer Ziel-DNA enzymatisch eine 5'-NH2-Funktionalisierung durchgeführt.

Aufgabe der vorliegenden Erfindung ist es, ein Verfahren zu schaffen, welche die Nachteile des Standes der Technik überwindet und in der Lage ist, effektiv und hochparallelisiert Cytosin-Methylierungen in einem Array von immobilisierten genomischen DNA-Proben aufzuzeigen.

Gegenstand der vorliegenden Erfindung ist daher ein Verfahren zur Auffindung von epigenetischen Informationsträgern in Form von 5-Methylcytosin-Basen in genomischer DNA, das eine Vielzahl von Sonden gleichzeitig zur massenspektrometrischen Untersuchung eines Arrays von Ziel-Nukleinsäuren verwendet.

Die erfindungsgemäße Aufgabe wird gelöst, in dem ein Verfahren Verfahren zur Identifikation von Cytosin-Methylierungsmustern in genomischen DNA-Proben zur Verfügung gestellt wird, bei dem man:
a) eine genomische DNA-Probe chemisch derart behandelt, daß Cytosin und 5-Methylcytosin unterschiedlich reagieren und man in der Duplex ein unterschiedliches Basenpaarungsverhalten der beiden Produkte erhält;
b) Teile der so behandelten DNA-Probe enzymatisch amplifiziert;
c) die amplifizierten Teile der so behandelten DNA-Probe an eine Oberfläche bindet;
d) einen Satz von Sonden unterschiedlicher Nukleobasensequenzen, die jeweils mindestens einmal die Dinukleotidsequenz 5'-CpG-3' enthalten, an die immobilisierten DNA-Proben hybridisiert;
e) die nicht hybridisierten Sonden abtrennt;
f) die hybridisierten Sonden in einem Massenspektrometer analysiert, wobei die Position der Sonden auf dem Probenträger eine Zuordnung zu der hybridisierenden DNA-Probe erlaubt;
g) Übertragung der aus den Massenspektren erhaltenen Peakmuster in Methylierungsmuster und Abgleich der neuen Daten mit einer Datenbank.

Erfindungsgemäß bevorzugt ist es, daß man in c) ein oder mehrere amplifizierte genomische DNA-Fragmente durch Hybridisierung mit komplementären Oligonukleotid- oder PNA-Sequenzen, die kovalent an die Oberfläche gebunden sind, immobilisiert.

Erfindungsgemäß bevorzugt ist es ferner, daß nach der Hybridisierung ein Cross-Linking der genomischen DNA-Fragmente mit den an der Oberfläche gebundenen Oligonukleotid- oder PNA-Sequenzen erfolgt. Besonders bevorzugt ist hierbei, daß man zum Cross-Linking kovalente chemische Bindungen ausbildet. Erfindungsgemäß bevorzugt ist hierbei ferner, daß man zum Cross-Linking elektrostatische Wechselwirkungen ausbildet.

Vorteilhaft ist es ferner, daß die an die Oberfläche gebundenen Oligonukleotid- oder PNA-Sequenzen 5-Bromuracil-Bausteine enthalten.

Erfindungsgemäß bevorzugt ist es, daß die immoblisierten komplementären Oligonukleotidsequenzen modifizierte Basen, Ribose oder Rückgrateinheiten beinhalten.

Das erfindungsgemäße Verfahren ist ferner dadurch gekennzeichnet, daß man in b) die genomische DNA-Probe in Form mehrerer amplifizierter Fragmente vermehrt, so daß man mindestens 0,01 % des gesamten Genoms amplifiziert.

Erfindungsgemäß bevorzugt ist es auch, daß man die Mischung amplifizierter DNA-Fragmente auf eine Oberfläche bindet, auf der eine Vielzahl von unterschiedlichen Punkten angeordnet ist die jeweils in der Lage sind, unterschiedliche Teile der amplifizierten DNA-Probe zu binden.

Erfindungsgemäß bevorzugt ist ferner, daß man in d) einen Satz von Sonden verwendet, welcher die Dinukleotid-Sequenz 5'-CpG-3' nur einmal je Sonde enthält und die Sonden ansonsten jeweils entweder keine Cytosin oder keine Guanin Basen umfassen.

Außerdem ist es erfindungsgemäß bevorzugt, daß man Schritt a) eine Bisulfit- oder Pyrosulfit- oder Disulfitlösung oder eine Mischung aus den angegebenen Lösungen, zusammen mit anderen Reagenzien für die spezifische oder hinreichend selektive Umwandlung von Cytosin in Uracil verwendet.

Vorteilhaft ist es auch, daß die zur Immobilisierung von amplifizierter Proben-DNA verwendete Oberfläche zugleich der Probenträger für ein Massenspektrometer ist. Bevorzugt ist es dabei, daß man die zur Immobilisierung von amplifizierter Proben-DNA verwendete Oberfläche als ganzes vor f) auf einen Probenträger für ein Massenspektrometer aufbringt. Bevorzugt ist es hierbei auch, daß man die hybridisierten Sonden vor dem, nach dem oder durch den Kontakt mit einer Matrix von den immobilisierten amplifizierten DNA-Proben ablöst.

Erfindungsgemäß bevorzugt ist es ferner, daß die Sonden Nukleinsäuren sind, die ein oder mehrere Massen-Tags tragen. Erfindungsgemäß vorteilhaft ist es auch, daß ein oder mehrere Massen-Tags zugleich Ladungs-Tags sind. Oder daß die Sonden zusätzlich ein Ladungs-Tag tragen.

Erfindungsgemäß bevorzugt ist es, daß die Sonden modifizierte Nukleinsäuremoleküle sind. Hierbei ist besonders bevorzugt, daß die modifizierten Nukleinsäuremoleküle PNAs, alkylierte Phosphorothioatnukleinsäuren oder Alkylphosphonatnukleinsäuren sind.

Erfindungsgemäß bevorzugt ist es, daß man die Sonden durch kombinatorische Synthese herstellt. Dabei ist erfindungsgemäß besonders bevorzugt, daß man unterschiedliche Basenbausteine derart markiert, daß die aus ihnen synthetisierten Sonden jeweils über ihre Massen im Massenspektrometer unterscheidbar sind.

Erfindungsgemäß vorteilhaft ist es ferner, daß man die Sonden als Teilbibliotheken herstellt und diese mit unterschiedlichen Massen- und/oder Ladungs-Tags versieht.

Ganz besonders erfindungsgemäß bevorzugt ist es, daß man in f) matrix-assistierte Laser Desorptions/Ionisations Massenspektrometrie (MALDI) ausführt.

Ein weiterer Gegenstand der vorliegenden Erfindung ist ein Kit für die Durchführung des erfindungsgemäßen Verfahrens, umfassend einen Probenträger für ein Massenspektrometer, welcher derart modifiziert ist, daß beliebig wählbare Teile eines Genoms an diesen immobilisiert werden und/oder Sondenbibliotheken, mit denen man die an den Probenträger immobilisierte DNA massenspektrometrisch analysiert und/oder weiteren Chemikalien, Lösungsmitteln und/oder Hilfsstoffen sowie gegebenenfalls einer Bedienungsanleitung.

Das erfindungsgemäße Verfahren dient zur Identifikation von 5-Methylcytosin-Positionen in genomischer DNA, die unterschiedlichsten Ursprungs sein kann. Die genomische DNA wird zunächst chemisch so behandelt, daß sich ein Unterschied in der Reaktion der Cytosin-Basen zu den 5-Methylcytosin-Basen ergibt. Mögliche Reagenzien sind hier z. B. Disulfit (auch als Bisulfit bezeichnet), Hydrazin und Permanganat. In einer bevorzugten Variante des Verfahrens wird die genomische DNA mit Disulfit in Gegenwart von Hydrochinon oder Hydrochinonderivaten behandelt, wobei selektiv nach anschließender alkalischer Hydrolyse die Cytosin-Basen in Uracil umgewandelt werden. 5-Methylcytosin bleibt unter diesen Bedingungen unverändert. Nach einem Aufreinigungsprozeß, der der Abtrennung des überschüssigen Disulfits dient, werden bestimmte Abschnitte der vorbehandelten genomischen DNA in einer Polymerasereaktion amplifiziert. In einer bevorzugten Variante des Verfahrens wird hier die Polymerasekettenreaktion eingesetzt. In einer besonders bevorzugten Variante des Verfahrens wird die Polymerasekettenreaktion so durchgeführt, daß mindestens 0.01% des gesamten Genoms in Form mehrerer Fragmente amplifiziert werden.

Die amplifizierte, vorbehandelte DNA-Probe kann nun in mehreren Varianten des Verfahrens an eine Oberfläche immobilisiert werden. In einer bevorzugten Variante des Verfahrens erfolgt die Immobilisierung an die Oberfläche derart, daß diese zuvor mit Oligonukleotiden oder kurzen PNA (Peptide nucleic acids) -Sequenzen modifiziert wurde und damit eine Hybridisierung komplementärer Sequenzen in der DNA-Probe erfolgt. Grundsätzlich können die immobilisierten Oligonukleotide sowohl an den Basen, an der (Desoxy)-Ribose und/oder am Rückgrat gegenüber herkömmlicher DNA modifiziert sein. Werden nun verschiedene Oligonukleotid- oder PNA-Sequenzen in Form eines Arrays an diese Oberfläche gebunden oder auf ihr synthetisiert, so kann jede dieser unterschiedlichen Sequenzen unterschiedliche Teile der amplifizierten DNA-Fragmente binden. In einer besonders bevorzugten Variante des Verfahrens wird im Anschluß an die Hybridisierung ein Cross-Linking der beiden Stränge durchgeführt. Die kann durch Ausbildung einer kovanten chemischen Bindung oder einer stabilen elektrostatischen Wechselwirkung erfolgen. In einer weiteren bevorzugten Variante wird ein photochemisches Cross-Linking über Bromuracil-Bausteine durchgeführt.

Auch ist es möglich, Fragmente der vorbehandelten genomischen DNA separat zu amplifizieren und die Produkte einzeln an unterschiedlichen Orten auf der Oberfläche zu immobilisieren. In einer bevorzugten Variante des Verfahrens erfolgt dies derart, daß einer der PCR-Primer eine zur Immobilisierung geeignete Funktion trägt, die mit einer auf der Oberfläche angebrachten Funktionalität eine Bindung eingehen kann.

Die Oberfläche, an die die amplifizierten DNA-Fragmente gebunden werden, soll sich entweder auf den Probenträger eines MALDI-Massenspektrometers übertragen lassen oder aber selbst dieser Probenträger sein. Die Konstruktion und Software des Massenspektrometers gewährleistet dabei, daß der jeweils untersuchte Punkt auf dem Probenträger den dort ursprünglich gebundenen Proben zugeordnet werden kann.

An die immobilisierten amplifizierten DNA-Fragmente wird nun ein Satz von Sonden hybridisiert, wobei diese Sonden je wenigstens einmal die Sequenz 5'-CpG-3' und ansonsten jeweils entweder keine Cytosin oder Guanin Basen enthalten. Die Sonden können Oligonukleotide, modifizierte Oligonu'kleotide oder PNAs (Peptide nucleic acids) sein. In einer bevorzugten Variante des Verfahren wird dieser Satz von Sonden in einem kombinatorischen Syntheseansatz als kombinatorische Bibliothek hergestellt. In einer weiteren bevorzugten Variante des Verfahrens sind die Sonden über ihre Massen eindeutig unterscheidbar, so daß ein Rückschluß von der Masse auf die Sequenz möglich ist. Dazu können die Sonden mit Massentags versehen sein, die die Massengleichkeit unterschiedlicher Sonden verhindern. Die Sonden können mit Ladungstags versehen sein, um eine bessere Darstellbarkeit im Massenspektometer zu erreichen und um die Stabilität der Analyse in Anwesenheit von Salzen und Detergenzien zu erhöhen. Die Massentags können zugleich Ladungstags sein. Die Sonden können auch als kombinatorische Teilbiblioteken hergestellt werden, die wiederum unterschiedliche Massen- und/oder Ladungstags tragen. Die Sonden können PNAs, unmodifizierte Nukeinsäuremoleküle oder modifizierte Nukleinsäuremoleküle wie Phosphothioatnukleinsäuren, alkylierte Phosphorothioatnukleinsäuren oder Alkylphopshonatnukleinsäuren sein, unbeschadet einer weiteren Modifikation durch Massen- und Ladungstags.

Die nicht hybridisierten Sonden werden in einem oder mehreren Waschschritten abgetrennt. Die hybridisierten Sonden bleiben dabei an ihren Positionen.

Die Oberfläche wird auf dem MALDI-Probenträger befestigt und in das Massenspektrometer transferiert bzw. unmittelbar transferiert, wenn das Verfahren auf dem MALDI-Probenträger selbst durchgeführt wurde. Der Array von Proben wird nun auf hybridisierte Sonden hin massenspektrometrisch untersucht. Die hybridisierten Sonden werden dazu in einer bevorzugten Variante des Verfahren durch den Kontakt mit der MALDI-Matrix dehybridisiert und in diese eingebettet; durch die Geschwindigkeit in der die Matrix aufgebracht wird erfolgt aber keine gegenseitige Kontamination der benachbarten Punkte. An jedem Punkt ergeben die hybridisierten Sonden ein Peakmuster, über das auf die Sequenzen geschlossen werden kann, an denen eine Hybridisierung erfolgte. Aufgrund der Vorbehandlung (vorzugsweise mit Bisulfit) ergeben unterschiedlich am Cytosin methylierte DNA-Fragmente unterschiedliche Sequenzen. Daher sind die durch die Sonden erzeugten Peakmuster im Massenspektrometer charakteristische Methylierungsmuster der jeweils untersuchten DNA-Probe. Es erfolgt ein Abgleich dieser Methylierungsmuster mit denen einer Datenbank.

## Patentansprüche

1. Verfahren zur Identifikation von Cytosin-Methylierungsmustern in genomischen DNA-Proben **dadurch gekennzeichnet, daß** man:
a) eine genomische DNA-Probe chemisch derart behandelt, daß Cytosin und 5-Methylcytosin unterschiedlich reagieren und man in der Duplex ein unterschiedliches Basenpaarungsverhalten der beiden Produkte erhält;
b) Teile der so behandelten DNA-Probe enzymatisch amplifiziert;
c) die amplifizierten Teile der so behandelten DNA-Probe an eine Oberfläche bindet;
d) einen Satz von Sonden unterschiedlicher Nukleobasensequenzen, die jeweils mindestens einmal die Dinukleotidsequenz 5'-CpG-3' enthalten, an die immobilisierten DNA-Proben hybridisiert;
e) die nicht hybridisierten Sonden abtrennt;
f) die hybridisierten Sonden in einem Massenspektrometer analysiert, wobei die Position der Sonden auf dem Probenträger eine Zuordnung zu der hybridisierenden DNA-Probe erlaubt;
g) Übertragung der aus den Massenspektren erhaltenen Peakmuster in Methylierungsmuster und Abgleich der neuen Daten mit einer Datenbank.

2. Verfahren nach Anspruch 1, **dadurch gekennzeichnet, daß** man in c) ein oder mehrere amplifizierte genomische DNA-Fragmente durch Hybridisierung mit komplementären Oligonukleotid- oder PNA-Sequenzen, die kovalent an die Oberfläche gebunden sind, immobilisiert.

3. Verfahren nach Anspruch 2, **dadurch gekennzeichnet, daß** nach der Hybridisierung ein Cross-Linking der genomischen DNA-Fragmente mit den an der Oberfläche gebundenen Oligonukleotid- oder PNA-Sequenzen erfolgt.

4. Verfahren nach Anspruch 3, **dadurch gekennzeichnet, daß** man zum Cross-Linking kovalente chemische Bindungen ausbildet.

5. Verfahren nach Anspruch 3, **dadurch gekennzeichnet, daß** man zum Cross-Linking elektrostatische Wechselwirkungen ausbildet.

6. Verfahren nach einem der Ansprüche 3 bis 5, **dadurch gekennzeichnet, daß** die an die Oberfläche gebundenen Oligonukleotid- oder PNA-Sequenzen 5-Bromuracil-Bausteine enthalten.

7. Verfahren nach mindestens einem der voranstehenden Ansprüche, **dadurch gekennzeichnet, daß** die immoblisierten komplementären Oligonukleotidsequenzen modifizierte Basen, Ribose oder Rückgrateinheiten beinhalten.

8. Verfahren nach einem der voranstehenden Ansprüche, **dadurch gekennzeichnet, daß** man in b) die genomische DNA-Probe in Form mehrerer amplifizierter Fragmente vermehrt, wobei man mindestens 0,01 % des gesamten Genoms amplifiziert.

9. Verfahren nach mindestens einem der voranstehenden Ansprüche, **dadurch gekennzeichnet, daß** man die Mischung amplifizierter DNA-Fragmente auf eine Oberfläche bindet, auf der eine Vielzahl von unterschiedlichen Punkten angeordnet ist die jeweils in der Lage sind, unterschiedliche Teile der amplifizierten DNA-Probe zu binden.

10. Verfahren nach einem der voranstehenden Ansprüche, **dadurch gekennzeichnet, daß** man in d) einen Satz von Sonden verwendet, welcher die Dinukleotid-Sequenz 5'-CpG-3' nur einmal je Sonde enthält und die Sonden ansonsten jeweils entweder keine Cytosin oder keine Guanin Basen enthalten.

11. Verfahren nach einem der voranstehenden Ansprüche, **dadurch gekennzeichnet, daß** man Schritt a) eine Bisulfit- oder Pyrosulfit- oder Disulfitlösung oder eine Mischung aus den angegebenen Lösungen, zusammen mit anderen Reagenzien für die spezifische oder hinreichend selektive Umwandlung von Cytosin in Uracil verwendet.

12. Verfahren nach einem der voranstehenden Ansprüche, **dadurch gekennzeichnet, daß** die zur Immobilisierung von amplifizierter Proben-DNA verwendete Oberfläche zugleich der Probenträger für ein Massenspektrometer ist.

13. Verfahren nach mindestens einem der Ansprüche 1 bis 11, **dadurch gekennzeichnet, daß** man die zur Immobilisierung von amplifizierter Proben-DNA verwendete Oberfläche als ganzes vor f) auf einen Probenträger für ein Massenspektrometer aufbringt.

14. Verfahren nach einem der Ansprüche 1 bis 13, **dadurch gekennzeichnet, daß** man die hybridisierten Sonden vor dem, nach dem oder durch den Kontakt mit einer Matrix von den immobilisierten amplifizierten DNA-Proben ablöst.

15. Verfahren nach einem der voranstehenden Ansprüche, **dadurch gekennzeichnet, daß** die Sonden Nukleinsäuren sind, die ein oder mehrere Massen-Tags tragen.

16. Verfahren nach Anspruch 15, **dadurch gekennzeichnet, daß** ein oder mehrere Massen-Tags zugleich Ladungs-Tags sind.

17. Verfahren nach Anspruch 15, **dadurch gekennzeichnet, daß** die Sonden zusätzlich ein Ladungs-Tag tragen.

18. Verfahren nach einem der voranstehenden Ansprüche, **dadurch gekennzeichnet, daß** die Sonden modifizierte Nukleinsäuremoleküle sind.

19. Verfahren nach Anspruch 20, **dadurch gekennzeichnet, daß** die modifizierten Nukleinsäuremoleküle PNAs, alkylierte Phosphorothioatnukleinsäuren oder Alkylphosphonatnukleinsäuren sind.

20. Verfahren nach einem der voranstehenden Ansprüche, **dadurch gekennzeichnet, daß** man die Sonden durch kombinatorische Synthese herstellt.

21. Verfahren nach Anspruch 20, **dadurch gekennzeichnet, daß** man unterschiedliche Basenbausteine derart markiert, daß die aus ihnen synthetisierten Sonden jeweils über ihre Massen im Massenspektrometer unterscheidbar sind.

22. Verfahren nach einem der voranstehenden Ansprüche, **dadurch gekennzeichnet, daß** man die Sonden als Teilbibliotheken herstellt und diese mit unterschiedlichen Massen- und/oder Ladungs-Tags versieht.

23. Verfahren nach einem der voranstehenden Ansprüche, **dadurch gekennzeichnet, daß** man in f) matrix-assistierte Laser Desorptions/Ionisations Massenspektrometrie (MALDI) ausführt.

## Claims

1. Method for the identification of cytosine methylation patterns in genomic DNA samples **characterized in that**:
a) a genomic DNA sample is treated chemically in such a way that cytosine and 5-methylcytosine react differently and a different base pairing behavior of the two products is obtained in the duplex;
b) portions of the thus-treated DNA sample are enzymatically amplified;
c) the amplified portions of the thus-treated DNA sample are bound to a surface;
d) a set of probes of different nucleobase sequences, each of which contains the dinucleotide sequence 5'-CpG-3' at least once, are hybridized to the immobilized DNA samples;
e) the non-hybridized probes are separated;
f) the hybridized probes are analyzed in a mass spectrometer, wherein the position of the probes on the sample holder permits a classification of the hybridizing DNA sample;
g) assignment of the peak pattern obtained from the mass spectra to the methylation pattern and comparison of the new data with a database.

2. Method according to claim 1, further **characterized in that** one or more amplified genomic DNA fragments are immobilized in c) by hybridization with complementary oligonucleotide or PNA sequences, which are covalently bound to the surface.

3. Method according to claim 2, further **characterized in that** a cross-linking of the genomic DNA fragments with the oligonucleotide or PNA sequences bound to the surface results after the hybridization.

4. Method according to claim 3, further **characterized in that** covalent chemical bonds are formed for the cross-linking.

5. Method according to claim 3, further **characterized in that** electrostatic interactions are formed for the cross-linking.

6. Method according to one of claims 3 to 5, further **characterized in that** the oligonucleotide or PNA sequences bound to the surface contain 5-bromouracil structural units.

7. Method according to at least one of the preceding claims, further **characterized in that** the immobilized complementary oligonucleotide sequences contain modified bases, ribose or backbone units.

8. Method according to one of the preceding claims, further **characterized in that** the genomic DNA sample is propagated in b) in the form of several amplified fragments, whereby at least 0.01% of the total genome is amplified.

9. Method according to at least one of the preceding claims, further **characterized in that** the mixture of amplified DNA fragments is bound to a surface, on which a multiple number of different points is arranged, each of which can bind different portions of the amplified DNA sample.

10. Method according to one of the preceding claims, further **characterized in that** a set of probes is used in d), which contains the dinucleotide sequence 5'-CpG-3' only once in each probe and the probes otherwise contain either no cytosine or no guanine bases.

11. Method according to one of the preceding claims, further **characterized in that** a bisulfite or pyrosulfite or disulfite solution or a mixture of the indicated solutions is used together with other reagents for the specific or sufficiently selective conversion of cytosine to uracil.

12. Method according to one of the preceding claims, further **characterized in that** the surface used for the immobilization of amplified sample DNA is also the sample holder for a mass spectrometer.

13. Method according to at least one of claims 1 to 11, further **characterized in that** the surface used for the immobilization of amplified sample DNA is introduced as a whole, prior to f), onto a sample holder for a mass spectrometer.

14. Method according to one of claims 1 to 13, further **characterized in that** the hybridized probes are stripped from the immobilized amplified DNA samples before, after or by contact with a matrix.

15. Method according to one of the preceding claims, further **characterized in that** the probes are nucleic acids, which bear one or more mass tags.

16. Method according to claim 15, further **characterized in that** one or more mass tags are also charge tags.

17. Method according to claim 15, further **characterized in that** the probes also bear a charge tag.

18. Method according to one of the preceding claims, further **characterized in that** the probes are modified nucleic acid molecules.

19. Method according to claim 20, further **characterized in that** the modified nucleic acid molecules are PNAs, alkylated phosphorothioate nucleic acids or alkyl phosphonate nucleic acids.

20. Method according to one of the preceding claims, further **characterized in that** the probes are prepared by combinatory synthesis.

21. Method according to claim 20, further **characterized in that** different base structural units are labeled in such a way that the each of the probes synthesized from them can be distinguished by their mass in the mass spectrometer.

22. Method according to one of the preceding claims, further **characterized in that** the probes are prepared as sublibraries and these are provided with different mass and/or charge tags.

23. Method according to one of the preceding claims, further **characterized in that** matrix-assisted laser desorption/ionization mass spectrometry (MALDI) is conducted in f).

## Revendications

1. Procédé d'identification de modèles de méthylation de cytosine dans des échantillons d'ADN génomique **caractérisé par le fait que** l'on :
a) traite chimiquement un échantillon d'ADN, de façon que la cytosine et la 5-méthylcytosine réagissent différemment et que l'on obtienne dans le duplex un comportement différent d'appariement de bases des deux produits ;
b) amplifie des fragments de l'échantillon d'ADN ainsi traité par voie enzymatique ;
c) fixe sur une surface les fragments amplifiés de l'échantillon d'ADN ainsi traité ;
d) hybride un kit de sondes de différentes séquences de nucléobases, contenant chacune au moins une fois la séquence de dinucléotide 5'-CpG-3', avec les échantillons d'ADN immobilisés ;
e) sépare les sondes non hybridées ;
f) analyse les sondes hybridées dans un spectromètre de masse, la position des sondes sur le porte-échantillon permettant une attribution à l'échantillon d'ADN hybridant ;
g) transfère des modèles de pic obtenus dans les spectres de masse aux modèles de méthylation et harmonise des nouvelles données à l'aide d'une base de données.

2. Procédé selon la revendication 1, **caractérisé par le fait que** l'on immobilise en c) un ou plusieurs fragments d'ADN génomique amplifiés par hybridation à l'aide de séquences complémentaires d'oligonucléotides ou de PNA, fixées par liaison covalente à la surface.

3. Procédé selon la revendication 2, **caractérisé par le fait qu'**après l'hybridation est effectué un cross-linking des fragments d'ADN génomique avec les séquences d'oligonucléotides ou de PNA fixés à la surface.

4. Procédé selon la revendication 3, **caractérisé par le fait que** l'on forme des liaisons chimiques covalentes destinées au cross-linking.

5. Procédé selon la revendication 3, **caractérisé par le fait que** l'on forme des interactions électrostatiques destinées au cross-linking.

6. Procédé selon une des revendications 3 à 5, **caractérisé par le fait que** les séquences d'oligonucléotides ou de PNA fixées à la surface contiennent des éléments 5-bromuracile.

7. Procédé selon au moins une des revendications précédentes, **caractérisé par le fait que** les séquences d'oligonucléotides complémentaires immobilisées contiennent des bases modifiées, des riboses ou des unités de structure.

8. Procédé selon une des revendications précédentes, **caractérisé par le fait que** l'on multiplie en b) l'échantillon d'ADN génomique sous forme de plusieurs fragments amplifiés, en amplifiant au moins 0,01 % du génome total.

9. Procédé selon au moins une des revendications précédentes, **caractérisé par le fait que** l'on fixe le mélange de fragments amplifiés d'ADN sur une surface, sur laquelle sont disposées une grande quantité de points divers qui sont chacun en mesure de fixer différentes fractions de l'échantillon amplifié d'ADN.

10. Procédé selon une des revendications précédentes, **caractérisé par le fait que** l'on utilise en d) un kit de sondes qui ne contient la séquence de dinucléotide 5'-CpG-3' qu'une seule fois par sonde et que les sondes ne contiennent, par ailleurs, soit pas de cytosine soit pas de guanine.

11. Procédé selon une des revendications précédentes, **caractérisé par le fait que** l'on utilise dans l'étape a) une solution de bisulfite ou de pyrosulfite ou de disulfite ou un mélange des solutions indiquées, en même temps que d'autres réactifs pour la transformation spécifique ou suffisamment sélective de cytosine en uracile.

12. Procédé selon une des revendications précédentes, **caractérisé par le fait que** la surface utilisée pour l'immobilisation des échantillons d'ADN amplifiés est en même temps le porte-échantillon pour un spectromètre de masse.

13. Procédé selon au moins une des revendications 1 à 11, **caractérisé par le fait que** l'on place la totalité de la surface utilisée pour l'immobilisation des échantillons d'ADN amplifiés avant f) sur un porte-échantillon pour un spectromètre de masse.

14. Procédé selon une des revendications 1 à 13, **caractérisé par le fait que** l'on sépare les sondes hybridées des échantillons d'ADN immobilisés et amplifiés avant, après ou par le contact avec une matrice.

15. Procédé selon une des revendications précédentes, **caractérisé par le fait que** les sondes sont des acides nucléiques portant un ou plusieurs marqueurs de masse.

16. Procédé selon la revendication 15, **caractérisé par le fait qu'**un ou plusieurs marqueurs de masse sont en même temps des marqueurs de charge.

17. Procédé selon la revendication 15, **caractérisé par le fait que** les sondes portent en plus un marqueur de charge.

18. Procédé selon une des revendications précédentes, **caractérisé par le fait que** les sondes sont des molécules modifiées d'acide nucléique.

19. Procédé selon la revendication 20, **caractérisé par le fait que** les molécules modifiées d'acide nucléique sont des PNAs, des acides nucléiques phosphorothioates alkylés ou des acides nucléiques alkylphosphonates.

20. Procédé selon une des revendications précédentes, **caractérisé par le fait que** l'on fabrique les sondes par synthèse combinatoire.

21. Procédé selon la revendication 20, **caractérisé par le fait que** l'on marque différents éléments de base de façon que les sondes qui sont synthétisées à partir d'eux puissent être différenciées par leur masse dans le spectromètre de masse.

22. Procédé selon une des revendications précédentes, **caractérisé par le fait que** l'on fabrique les sondes sous forme de bibliothèques partielles et qu'on les munit de marqueurs différents de masse et de charge.

23. Procédé selon une des revendications précédentes, **caractérisé par le fait que** l'on exécute en f) une spectrométrie de masse par désorption-ionisation par laser assistée par matrice (MALDI).
